# EUROPEAN PATENT APPLICATION

(11) **EP 1 506 997 A1**
(43) Date of publication of application: **16.02.2005**
(21) Application number: 03017676.2
(22) Date of filing: 14.08.2003
(51) Int. Cl.: C12N 5/06, C12N 5/08

(54) **Method of generating neural stem cells**

(71) Applicant: NeuroProgen GmbH Leipzig, 04103 Leipzig (DE)
(72) Inventor: Schwarz, Sigrid, Dr., 04316 Leipzig (DE)
(74) Representative: Keller, Günter, Dr.

(57) **Abstract**

The present invention relates to a method of generating neural stem cells from mesodermal cells. The invention further provides a cell population which has neural stem cell-like characteristics. The neural stem cells prepared according to the invention are useful in the treatment of neurodegenerative disorders such as Alzheimer's disease and Parkinson's disease.

SEQUENCE LISTING

<110>  NeuroProgen GmbH Leipzig

<120>  Method of generating neural stem cells

<130>  method of generating neural stem cells

<160>  26

<170>  PatentIn version 3.1

<210>  1
<211>  20
<212>  DNA
<213>  Artificial Sequence

<220>
<223>  Primer

<400>  1
aagagctacg agctgcctga                                                20

<210>  2
<211>  20
<212>  DNA
<213>  Artificial Sequence

<220>
<223>  Primer

<400>  2
gctgatccac atctgctgga                                                20

<210>  3
<211>  22
<212>  DNA
<213>  Artificial Sequence

<220>

<223>  Primer

<400>  3
gagatcagtg ggataagcag ca                                                22

<210>  4
<211>  21
<212>  DNA
<213>  Artificial Sequence

<220>
<223>  Primer

<400>  4
cctcttcatg acgcttgtgg a                                                 21

<210>  5
<211>  20
<212>  DNA
<213>  Artificial Sequence

<220>
<223>  Primer

<400>  5
gaggcggcca gttatcagga                                                   20

<210>  6
<211>  20
<212>  DNA
<213>  Artificial Sequence

<220>
<223>  Primer

<400>  6
gttctcctcg ccctctagca                                                   20

<210> 7
<211> 20
<212> DNA
<213> Artificial Sequence

<220>
<223> Primer

<400> 7
ggatgggcaa ctgtacctga 20

<210> 8
<211> 21
<212> DNA
<213> Artificial Sequence

<220>
<223> Primer

<400> 8
tgttacgagc agtgatgcct a 21

<210> 9
<211> 21
<212> DNA
<213> Artificial Sequence

<220>
<223> Primer

<400> 9
tggctcagag gaagagtctg a 21

<210> 10
<211> 21
<212> DNA
<213> Artificial Sequence

<220>

<223>  Primer

<400>  10
tcccccattt acatgctgtg a                                                21

<210>  11
<211>  20
<212>  DNA
<213>  Artificial Sequence

<220>
<223>  Primer

<400>  11
aggactttca aaggccaagg                                                  20

<210>  12
<211>  21
<212>  DNA
<213>  Artificial Sequence

<220>
<223>  Primer

<400>  12
tcctccaaca tttgtcactt g                                                21

<210>  13
<211>  20
<212>  DNA
<213>  Artificial Sequence

<220>
<223>  Primer

<400>  13
agctcctgag cttgtccttg                                                  20

<210> 14
<211> 20
<212> DNA
<213> Artificial Sequence

<220>
<223> Primer

<400> 14
tgtccacgct gtactggttc 20

<210> 15
<211> 20
<212> DNA
<213> Artificial Sequence

<220>
<223> Primer

<400> 15
agtgatgagc atggcatcga 20

<210> 16
<211> 20
<212> DNA
<213> Artificial Sequence

<220>
<223> Primer

<400> 16
aggcagtcgc agttttcaca 20

<210> 17
<211> 20
<212> DNA
<213> Artificial Sequence

<220>

<223>  Primer

<400>  17
acctcgttga cacctggaag                                                    20

<210>  18
<211>  20
<212>  DNA
<213>  Artificial Sequence

<220>
<223>  Primer

<400>  18
ccaccatctc ggagagtgac                                                    20

<210>  19
<211>  21
<212>  DNA
<213>  Artificial Sequence

<220>
<223>  Primer

<400>  19
agctctgtgg ccatgggccc c                                                  21

<210>  20
<211>  22
<212>  DNA
<213>  Artificial Sequence

<220>
<223>  Primer

<400>  20
agacagacca actagaagat ga                                                 22

<210>  21
<211>  24
<212>  DNA
<213>  Artificial Sequence

<220>
<223>  Primer

<400>  21
cggagtcaac ggatttggtc gtat                                              24

<210>  22
<211>  25
<212>  DNA
<213>  Artificial Sequence

<220>
<223>  Primer

<400>  22
agccttctcc atggttggtg aagac                                             25

<210>  23
<211>  22
<212>  DNA
<213>  Artificial Sequence

<220>
<223>  Primer

<400>  23
taccagaccg agaccaacag ag                                                22

<210>  24
<211>  19
<212>  DNA
<213>  Artificial Sequence

<220>

<223>  Primer

<400>  24
caccaccggg tcacgtcgc                                                    19

<210>  25
<211>  20
<212>  DNA
<213>  Artificial Sequence

<220>
<223>  Primer

<400>  25
ctacgactgg acgctggtgc                                                   20

<210>  26
<211>  20
<212>  DNA
<213>  Artificial Sequence

<220>
<223>  Primer

<400>  26
cgatgtccac gtcgcggaag                                                   20

## Description

The present invention relates to a method of generating neural stem cells from mesodermal cells. The invention further provides a cell population which has neural stem cell-like characteristics. The neural stem cells prepared according to the invention are useful in the treatment of neurodegenerative disorders such as Alzheimer's disease and Parkinson's disease.

There are severe diseases associated with the degeneration or functional disorder of certain specific cells. Due to the rapid development of stem cell technology cell replacement therapies have opened interesting possibilities in the treatment of those disorders. Stem cells have the capacity to both self-renew and to generate a large number of differentiated progeny. Stem cells can be isolated from the developing embryo and from specific adult tissues, but their potential for differentiation into all cell types becomes restricted gradually to a more limited range of cells typical of the mature tissue in which the stem cell (or progenitor cell) resides.

Clonogenic neural stem cells (NSC) are self-renewing cells that maintain the capacity to differentiate into brain-specific cell types [Fricker et al., 1999; Kilpatrick & Bartlett, 1993; Uchida et al., 2000], and may also replace or repair diseased brain tissue [Kim et al., 2002; Pluchino et al., 2003; Carvey et al., 2001]. NSCs can be directly isolated from fetal or adult nervous tissue [Reynolds & Weiss, 1992; Kilpatrick & Bartlett, 1993; Cattaneo & McKay, 1990], or derived from embryonic stem cells [Bain et al., 1995; Lee et al., 2000].

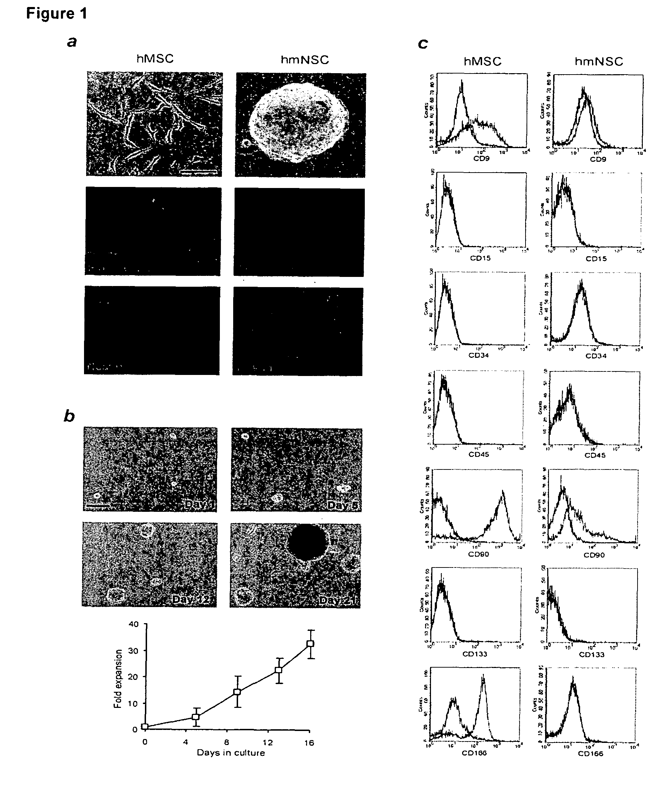

Bone marrow stromal cells (MSCs) grown out of bone marrow cell suspensions by their selective attachment to tissue culture plastic can be expanded efficiently (Figure 1a) [Friedenstein et al., 1975; Reyes et al., 2001; Sekiya et al., 2002]. MSCs are capable of differentiating into multiple mesodermal tissues, including bone, cartilage, fat, and muscle [Prockop, 1997, Sekiya et al., 2002; Reyes et al., 2002]. In addition, these cells can break barriers of germ layer commitment and differentiate in vitro and in vivo into cells expressing neuronal and glial markers [Woodbury et al., 2000; Sanchez-Ramos et al., 2000; Zhao et al., 2002].

However, differentiated neuronal cells are well known to poorly survive detachment and subsequent transplantation procedures. Accordingly, neural stem cells or premature neural cells are more suitable for neurotransplantation strategies compared to fully differentiated neural cells. There exists an ongoing need for cell sources that are suitable for neurotransplantations.

The inventors surprisingly found that MSCs can be converted into undifferentiated NSCs by culturing them in a culture medium containing EGF and FGF-2. The resulting cells can be proliferated and differentiated *in vitro* into glia and neurons.

The present invention therefore relates to a method of generating neural stem cells, comprising culturing mesenchymal stem cells in a medium containing EGF and FGF-2 wherein hepatocyte growth factor is not present in said medium.

As used herein, the term "mesenchymal stem cell" designates a cell which is capable of proliferating and differentiating into myogenic, osteogenic, chondrogenic, and adipogenic lineages. Mesenchymal stem cells may be derived from, e.g., bone marrow, umbilical cord blood or fat tissue. Usually, mesenchymal stem cells express the marker molecules CD10, CD13, CD61, CD90, CD140b, CD109, CD172a and CD105 (endoglin), whereas they do not express CD45, CD34, and CD133 [Vogel et al., 2003].

The mesenchymal cells can be isolated from bone marrow, preferably adult bone marrow, more preferably human adult bone marrow. Alternatively, the mesenchymal stem cells can be isolated from umbilical cord blood, preferably human umbilical cord blood, or from adipose tissue. Methods of isolating mesenchymal stem cells from various sources are known to those skilled in the art (see, e.g., Fiedler et al., 2002; Vogel et al., 2003; Ashjian et al., 2003; Erices et al., 2000; Romanov et al., 2003; Tholpady et al., 2003; Wickham et al., 2003).

The term "adult" preferably refers to an individual at an age of at least about 16 years, e.g., about 16 years to about 45 years, more preferably at least about 18 years, e.g., about 18 years to about 30 years. However, also individuals at an age of less than 16 years or greater than 45 years may be suitable donors.

As used herein, the term "neural stem cell" designates a cell which is capable of self-renewing and of differentiating into glial cells and neurons. Usually, neural stem cells express the marker molecules nestin, CD15, CD56, CD90, CD164, and NGFR, whereas they do not express CD45, CD105 (endoglin), CD109, and CD140b (PDGF-RB) [Vogel et al., 2003].

The term "self-renewing" refers to the capability of a cell to undergo mitosis and to divide to produce two daughter cells.

According to the invention mesenchymal stem cells are cultured in a medium containing EGF (=epidermal growth factor) and FGF-2 (=basic FGF=fibroblast growth factor 2). EGF and FGF-2 are commercially available. The concentration of EGF in the culture medium is preferably 0.2 ng/ml to 2 µg/ml, more preferably 2 ng/ml to 200 ng/ml, still more preferably 10 ng/ml to 100 ng/ml, most preferably about 20 ng/ml. The concentration of FGF-2 in the culture medium is preferably 0.2 ng/ml to 2 µg/ml, more preferably 2 ng/ml to 200 ng/ml, still more preferably 10 ng/ml to 100 ng/ml, most preferably about 20 ng/ml.

The mesenchymal stem cells may be cultured in the presence of EGF and FGF-2 for at least 2 days, preferably for at least 10 days, most preferably for at least 20 days, e.g., for 20 to 70 days. In one aspect, the mesenchymal stem cells are cultured in the presence of EGF and FGF-2 until neurosphere formation is observed, e.g., for 10 to 20 days. These neurospheres may be further expanded in medium containing EGF and FGF-2 for 1 to 15 weeks, preferably for 2 to 10 weeks.

In a particular embodiment of the invention, the mesenchymal cells are cultured under an atmosphere having an oxygen concentration of less than 20% (v/v). Preferably, the oxygen concentration is less than 15% (v/v), more preferably less than 10% (v/v), still more preferably less than 5% (v/v), most preferably about 3% (v/v). The minimum oxygen concentration may be 0.1% (v/v) or 1% (v/v). The cells may be cultured under an atmosphere of 5% (v/v) CO₂, 92% (v/v) N₂ and 3±2 % (v/v) O₂.

The cells may be cultured at a temperature of about 35-38°C, preferably at about 36-38°C, most preferably at about 37°C.

The cells may be cultured under conditions such that substantially no differentiation into neural cells is observed. This is accomplished by excluding from the culture medium agents that are known to induce neural differentiation such as hepatocyte growth factor (WO 02/086108 A1), brain-derived neurotrophic factor (BDNF), neurotrophine-3, nerve growth factor (NGF), glial cell line derived neurotrophic factor (GDNF), interleukin-1, interleukin-11, leukemia inhibitory factor (LIF), and the like.

The method of the invention may further comprise one or more of the following steps:
- following the culture in a medium containing EGF and FGF-2, the obtained mesodermal-derived neural stem cells can be enriched, e.g. by separating them from undesired cells which may be present as a minor fraction of the cells. Examples of such undesired cells include but are not limited to dead cells, remaining mesenchymal stem cells, fully differentiated neural cells and glial cells;
- the mesodermal-derived neural stem cells obtained by the method described supra may be cryopreserved according to an established protocol (Bruder et al., 1997; De Bari et al., 2001);
- the mesodermal-derived neural stem cells obtained by the method described supra may be further differentiated into neurons or glial cells by protocols known in the art. In this embodiment, the neural stem cells are further cultured in a medium containing a suitable differentiation inducing agent. For glial induction, PDGF may be used, for neuronal induction, BDNF may be used. Either of both growth factors may be combined with retinoic acid. Differentiation usually takes 7 to 20 days, preferably 10 to 14 days. Preferably, EGF and FGF-2 are not present during differentiation into glia or neurons.
- Neural stem cells may be enriched or depleted for a certain cell type. For example oligodendrocytes may be selected using an antibody directed against O4 and cell sorting (MACS or FACS).
- Prior to culturing the mesenchymal stem cells, the cells may be depleted or enriched for a certain cell type. For example, hematopoietic cells may be depleted or mononuclear cells may be isolated. Bone marrow stromal cells may be enriched by selective attachment to tissue culture plastic.

In a specific embodiment, no enrichment and/or isolation of mononuclear cells is performed prior to culturing the mesenchymal stem cells in the presence of EGF and FGF-2.

Another aspect of the invention is a method for making a medicament comprising the steps of (a) generating neural stem cells using the method described supra; and (b) adding to the neural stem cells a pharmaceutically acceptable carrier or diluent. Suitable carriers or diluents include, but are not limited to, physiological electrolyte solutions, e.g., sodium chloride solutions.

In another aspect the invention concerns a population of mesodermal-derived neural stem cells obtainable by a method described supra. The cells obtainable by the method according to the invention exhibit properties similar to those of neural stem cells. They usually lack expression of the marker molecules CD15, CD34, CD133 and CD166. The expression of marker molecules can be determined by FACS analysis as described in the Examples.

The cells obtained according to the invention express *NES* (encoding nestin) and *FN1* (encoding fibronectin). The amount of expression of a particular gene can be determined by quantitative real-time RT-PCR as described in the examples.

The amount of *NES* expression in the population of mesodermal-derived neural stem cells is significantly higher than that in the starting population of mesenchymal stem cells. Preferably, the amount of *NES* expression is increased by at least 100%, more preferably by at least 200%, most preferably by at least 300%, compared to the population of mesenchymal stem cells.

The amount of *FN1* expression in the population of mesodermal-derived neural stem cells is about 50% to about 150% compared to the amount of *FN1* expression in the starting population of mesenchymal stem cells. Preferably, the amount of *FN1* expression in the population of mesodermal-derived neural stem cells is about 80% to about 120% compared to the amount of *FN1* expression in the starting population of mesenchymal stem cells.

The mesodermal-derived neural stem cells of the invention express only low amounts of the glial marker gene *GFAP,* and of the neuronal marker genes *TUBB4*/*III* and *SNCA.* Usually, the amount of expression of GFAP is less than about 50%, preferably less than about 25% of that in fully differentiated glial cells, e.g., astrocytes. Usually, the amount of expression of *TUBB4*/*III* is less than about 50%, preferably less than about 25%, more preferably less than about 15% of that in fully differentiated neuronal cells, e.g., dopaminergic neuronal cells. Usually, the amount of expression of *SNCA* is less than about 50%, preferably less than about 25%, more preferably less than about 15% of that in fully differentiated neuronal cells.

Another aspect of the invention is the use of a population of mesodermal-derived neural stem cells according to the invention for the manufacture of a medicament for the treatment of a neurological disorder. Neurological disorders include, but are not limited to disorders with substantial loss of neurons and/or glial cells, e. g., stroke, multiple sclerosis, Alzheimer's disease, Parkinson's disease.

The cells obtained according to the process disclosed herein may be administered to a patient in need thereof using an established transplantation protocol. Such administration may be carried out by a method known in the art, such as surgery, with an infusion cannula, needle, or the like.

The number of mesodermal-derived neural stem cells to be administered ranges from 100 to 100,000,000, preferably 1,000 to 10,000,000, more preferably 10,000 to 10,000,000, most preferably 100,000 to 10,000,000 cells. Usually 100 to 10,000,000, preferably 1,000 to 10,000,000, more preferably 10,000 to 10,000,000, still more preferably 100,000 to 5,000,000, most preferably 1,000,000 to 5,000,000 cells are administered in a volume of 1 to 100 µl. The cells may be administered once or several times (e.g., twice, two times, three times, four times, five times, six times, seven times, eight times, nine times or ten times) at intervals of 2 to 24 weeks.

Another aspect of the invention is the use of mesenchymal stem cells for the manufacture of a medicament for the treatment of a neurological disorder. According to this embodiment, the mesenchymal stem cells are first cultured *in vitro* in the presence of EGF and FGF-2 (in the absence of hepatocyte growth factor). The mesodermal-derived neural stem cells thus obtained may be combined with a pharmaceutically acceptable carrier or diluent. Subsequently, the cells may be administered to a patient in need thereof in order to ameliorate the condition to be treated.

The cells administered to the patient usually are human cells. Preferably, the cells administered to the patient are autologous cells.

The invention further pertains to a method for treating a neurological disorder in an individual, which comprises administering to the individual a therapeutically effective amount of the mesodermal-derived neuronal stem cells described herein. The preferred embodiments of this method correspond to the preferred embodiments described above.

The present invention pertains to the efficient conversion of human adult bone marrow stromal cells (MSC) [Friedenstein et al., 1976; Prockop, 1997; Reyes et al., 2002] into a neural stem-like cell population (hMNSC, for human mesodermal-derived NSC). These cells grow in neurospheres, express high levels of the NSC marker nestin, but loose characteristics of mesodermal stromal cells. In the presence of selected growth factors, hMNSCs can be differentiated into the three main neural phenotypes: astroglia, oligodendroglia and neurons. Clonal analysis demonstrates that individual hMNSCs are multipotent and retain the capacity to generate both glia and neurons. The cell culture system described herein provides a powerful tool for investigating the molecular mechanisms of neural differentiation in adult human NSCs. hMNSCs may ultimately help to treat acute and chronic neurodegenerative diseases.
Figure 1 shows the characteristics of adult hMSC and human mesodermal-derived neural stem cells (hMNSC) during expansion. a, Morphology, fibronectin and nestin expression of hMSC (left panel) and hMNSC (right panel). All hMSCs express high levels of fibronectin, but only ≈ 5% of cells express very low levels of the NSC marker nestin, while some hMNSCs express low levels of fibronectin, but all cells express high levels of nestin. Nuclei are counterstained with DAPI (blue). Scale bars, 100 µm. **b**, Representative sequence of phase contrast microphotographs of hMNSCs after 1, 5, 12, and 21 days after conversion from hMSCs. Growth curve of hMNSCs revealed by enumerating the cells at each time point under a hemocytometer (n=5; calculated doubling time = 2.6 days). Scale bars, 50 µm. **c**, hMSCs (left panel) and hMNSCs (right panel) cultured for 10-50 and 5-30 population doublings, respectively, were labeled with FITC-coupled antibodies against CD9, CD15, CD34, CD45, CD90, CD133, CD166 or immunoglobulin isotype control antibodies. Cell were analyzed using a FACSCalibur flow cytometer. Black line, control immunoglobulin; red line, specific antibody.

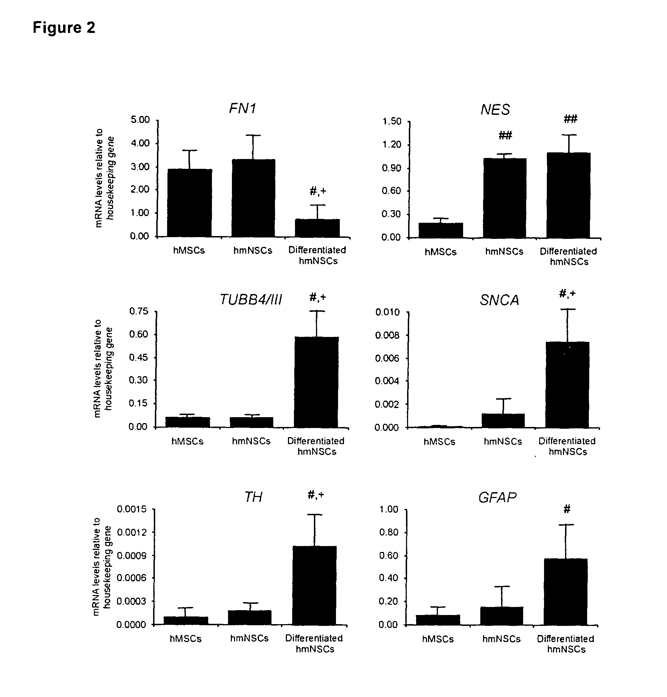

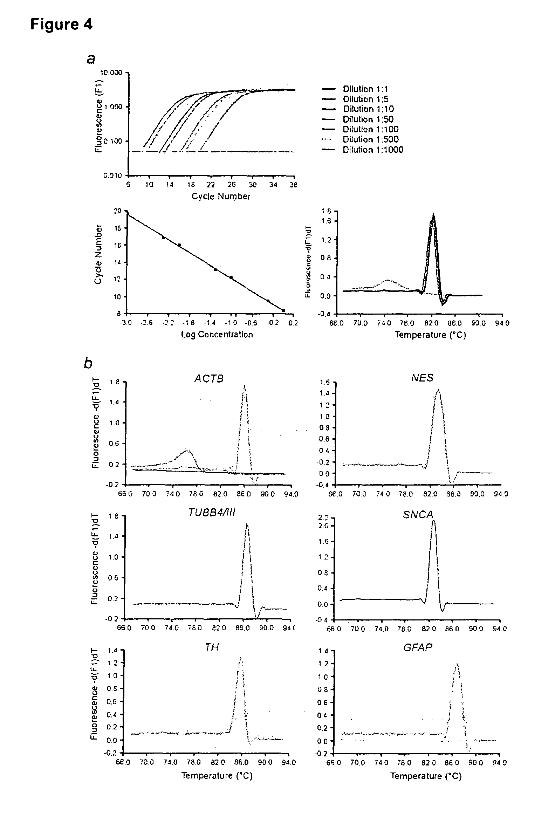

Figure 2 shows the Gene expression profile of hMSC, hMNSCs and differentiated hMNSCs into glial and neuronal cell types using the neuronal induction protocol for 14 days. Quantitative real-time RT-PCR for *FN1* (encoding for fibronectin), *NES* (nestin), *GFAP* (GFAP), *TUBB4*/*III* (β-tubulin III), *SNCA* (α-synuclein) and *TH* (TH) was performed using the LightCycler® technique and expression levels are expressed relative to the housekeeping gene *ACTB* (*β*-actin) or *HMBS* (porphobilinogen deaminase). For primers see Table 1, for representative standard curves and melting curve analysis demonstrating the specificity of amplified PCR products, see Figure 4. # indicates *P* < 0.05, ## represents *P* < 0.01 when compared to hMSCs; + indicates *P* < 0.05 when compared to hMNSCs.

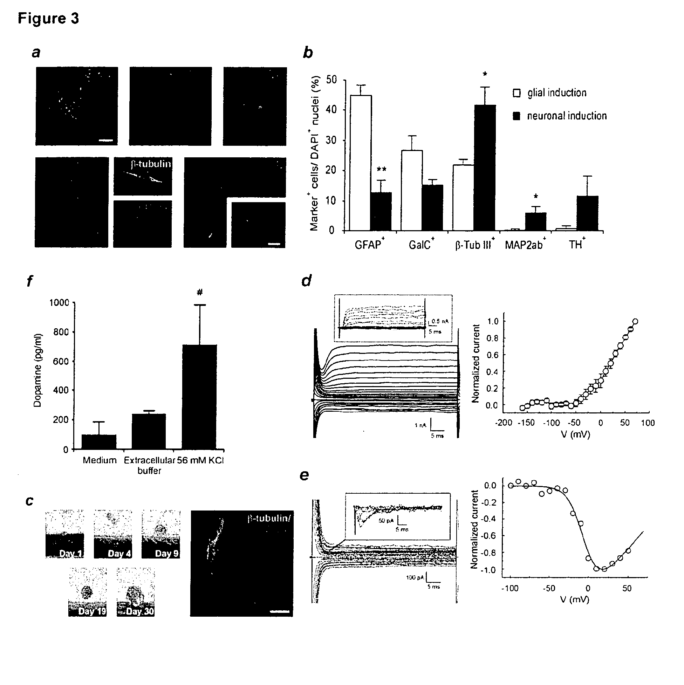

Figure 3 shows the *In vitro* differentiation of hMSC-derived neurospheres to astroglial, oligodendroglial and neuronal cell types. hMNSCs were differentiated after 5 to 30 population doublings using the glial induction or the neuronal induction protocol on poly-L-lysine for 14 days. **a**, Differentiated hMNSCs were stained for markers for astrocytes (GFAP), oligodendrocytes (GaIC), neurons (β-tubulin-III, MAP2ab), or catecholaminergic cells (TH), respectively. Nuclei are counterstained with DAPI (blue). Scale bars, 100 µm. **b,** Quantification of 14-day cultures of hMNSCs differentiated with the glial and the neuronal induction protocol, respectively. Data shown are mean values ± s.e.m. from at least three independent hMSC preparations. Significant differences from glial induction protocol are marked with a single (*P* < 0.05, *t*-test) or double (*P* < 0.01, *t*-test) asterisk. **c**, Clonal analysis of hMNSCs. Sequential phase contrast microphotographs of a hMNSC clone after 1, 4, 9, 19, 30 and 39 days after plating (upper panel). Progeny of single cell-derived neurospheres can be differentiated into neurons (β-tubulin III⁺, green) and astrocytes (GFAP⁺, red). Nuclei are counterstained with DAPI (blue). Scale bars, 100 µm. **d**, Electrophysiological recordings. For voltage-clamp measurements, cells were held at -80 mV and hyper- or depolarized in 10 mV steps between -160 and +70 mV. Example of a sustained outward current shown without and with (inset) leak subtraction using a -P/4 protocol. Current-voltage relationship of the normalized outward currents recorded with leak subtraction (n=7). **e**, Example of an inward current without and with leak subtraction (inset: only currents for depolarizing steps to -40, -20, 0, 20 and 60 mV are shown). Peak current-voltage relationship for the same cell with leak subtraction. The line represents a fit to the following equation: I(V)/Iₘₐₓ = g×(V-Vᵣₑᵥ)/(1+exp((V-V_{0.5})/k_{V}), with I (Iₘₐₓ) being the (maximum) membrane current, g the maximum conductance, V the applied voltage, Vᵣₑᵥ the reversal potential for Na⁺, V_{0.5} the potential of half-maximal activation and k_{V} a slope factor. **f**, Dopamine production and release were measured in hMNSCs differentiated using the neuronal induction protocol. The HPLC-ECD determination of dopamine concentration is shown in medium conditioned for 3 days (left), in extracellular buffer conditioned for 45 min. (center), and in extracellular buffer + 56 mM KCI conditioned for 45 min. (right). # indicates *P* < 0.05 when compared to extracellular buffer (paired *t*-test).
Figure 4 shows a real time PCR analysis of mesenchymal and neural genes on mRNA level. ***a***, Real-time PCR analysis of *FN1* mRNA encoding for fibronectin. Upper diagram: Plot of the fluorescence versus the cycle number obtained from SYBR Green detection of serially diluted *FN1* mRNA. The crossing line represents the position of the threshold. Lower diagrams show the standard curve (left) obtained by plotting cycle number of crossing points versus dilution factor as well as melting curve analysis (right). ***b***, Melting curve analyses of all other real-time PCR reactions used in the present study is performed to verify that the SYBR Green dye only detected the specific PCR product resulting in a single peak. In the *ACTB* mRNA analysis (upper left diagram) the melting point curve analyses of both the water (blue line) and the RT-minus sample (green line) are also included.

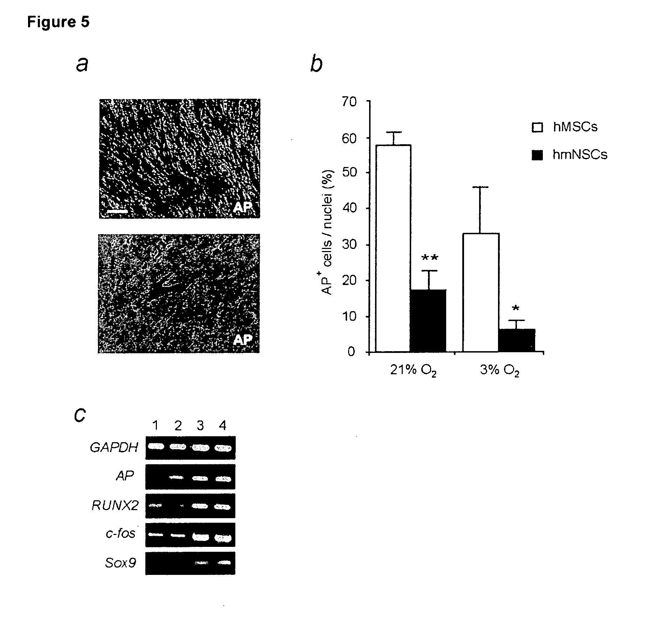

Figure 5 shows an analysis of the osteogenic differentiation ability of both hMSCs and hMNSCs. Both cells types were differentiated after 6 to 10 passages using the osteoblast differentiation protocol for 10 days (for details, see Supplementary Experimental Protocol). ***a***, Differentiated hMSCs (upper panel) and hMNSCs (lower panel) were stained for the osteogenic marker alkaline phosphatase (AP⁺). Scale bar, 50 µm. ***b***, Quantification of 10-day cultures of hMSCs and hMNSCs, respectively, differentiated into osteoblasts under normal atmospheric oxygen levels routinely used for osteoblast cultures (21%) and reduced atmospheric oxygen levels used in our neural system (3%). Data shown are mean values +/- s.e.m. from at least three independent cell preparations. Significant differences from hMSCs are marked with a single (*P*<0.05, *t*-test) or double (*P*<0.01, *t*-test) asterisk. **c**, Expression of osteogenic marker genes in hMSCs and hMNSCs after osteogenic differentiation under both normal and reduced atmospheric oxygen levels (21% and 3%, respectively). Semiquantitative RT-PCR analysis of alkaline phosphatase *(AP),* runt-related transcription factor 2 (*RUNX2*), and the transcription factors *Sox9* and *c-fos,* as well as *GAPDH* (housekeeping gene). Lane 1, hMNSC (21% O₂); lane 2, hMNSC (3% O₂); lane 3, hMSC (3% O₂); lane 4, hMSC (21% O₂).

The following non-limiting examples further illustrate the invention.

### Methods

**Cell culture.** Adult human bone marrow was harvested from routine surgical procedures (3 samples, age 18-30 years) with informed consent and in accordance with the terms of the ethics committee of the University of Ulm. hMSC were isolated and cultured as described earlier [Fiedler et al., 2002]. Cells were passaged once a week. The hMSC phenotype was proved by FACS analysis with CD9, CD90, CD105, and CD166 (positive), as well as CD14, CD34, and CD45 (negative), and by the potential to differentiate in osteoblasts, chondrocytes and adipocytes.

After passage 2 to 10 (≈ 10 to 50 population doublings) conversion of hMSC into neurospheres was initiated. Specifically, cells were dissociated by 0.05% trypsin/0.04% EDTA, and plated on tissue culture low-attachment plastic flasks at a concentration of 1.0-2.0×10⁵ cells cm⁻² in P4-8F medium (AthenaES, Baltimore, MD) supplemented with 20 ng ml⁻¹ of both EGF and FGF-2 (both from Sigma, St. Louis, MO) at 5% CO₂, 92% N₂ and 3±2% O₂. After 10 to 20 days, sphere formation could be observed. These neurospheres were expanded for additional 2 to 10 weeks (2 to 4 passages; ≈ 5 to 30 population doublings) before glial or neuronal differentiation was initiated. The medium was changed once a week while growth factors were added twice a week. Induction of neural differentiation was initiated by plating the cells on poly-L-lysin coated glass cover slips at a concentration of 1.5-2.0×10⁵ cells cm⁻² in NB medium (Gibco, Tulsa, OK) supplemented with 0.5 µmol I⁻¹ all-trans-retinoic acid (Sigma), and 1% FCS, 5% horse serum, 1% N2 supplement and 1% penicillin/streptomycin (all from Gibco), and 10 ng ml⁻¹ rh-PDGF-BB (glial induction; R&D Systems, Minneapolis, MN) or 10 ng ml⁻¹ rh-BDNF (neuronal induction; Promega, Madison, WI). Cells were differentiated for 10 to 14 days.

For clonal analysis, hMNSCs were serially diluted into the hMNSC expansion medium in 96-multiwell plates. Only single cells were expanded by supplementing the medium to 50% with filtered hMNSC conditioned medium containing growth factors. Cells were expanded for 3 to 6 weeks and further processed as described above for hMNSC using the glial induction protocol.

**Flow cytometry.** hMSC and hMNSC were treated with trypsin-EDTA (Gibco) and washed with PBS. Dead cells were excluded from analysis by forward-scatter gating. Samples were analyzed using FACSCalibur flow cytometer and Cellquest software (both from Becton Dickinson, Franklin Lakes, NJ). A minimum of 12,000 events was acquired for each sample.

**lmmunocytochemistry.** Cells were fixed in 4% paraformaldehyde in PBS. Immunocytochemistry was carried out using standard protocols. Cell nuclei were counter stained with 4,6-diamidino-2-phenylindole (DAPI). Antibodies and dilutions were as follows: TH monoclonal 1:1000, β-tubutin III monoclonal 1:1000, fibronectin monoclonal 1:400 (all from Sigma), MAP2ab monoclonal 1:300 and GFAP monoclonal 1:1000 (both Pharmingen, San Diego, CA), GaIC monoclonal 1:750, GFAP polyclonal 1:1000, nestin polyclonal 1:500 (all from Chemicon International, Temecula, USA), and fluorescence labeled secondary antibodies (Jackson, West Grove, PA).

**Quantitative real-time RT-PCR analysis.** Total cellular RNA was extracted from hMSCs, hMNSCs and differentiated hMNSCs (neuronal induction medium) using RNAeasy total RNA purification kit followed by treatment with RNase-free DNase (Qiagen, Hilden, Germany). Quantitative real-time one step RT-PCR was carried out using the LightCycler© System (Roche, Mannheim, Germany), and amplification was monitored and analyzed by measuring the binding of the fluorescence dye SYBR Green I to double-stranded DNA. 1 µl of total RNA was reversely transcribed and subsequently amplified using QuantiTect SYBR Green RT-PCR Master mix (Qiagen) and 0.5 µmol I⁻¹ of both sense and antisense primers. Tenfold dilutions of total RNA were used as external standards. Standards and samples were simultaneously amplified. After amplification, melting curves of the RT-PCR products were acquired to demonstrate product specificity (Figure 4). The results are expressed relative to the housekeeping genes *ACTB* (β-actin) or *HMBS* (porphobilinogen deaminase; PBGD). Primer sequences and product lengths products can be found in Table 1:

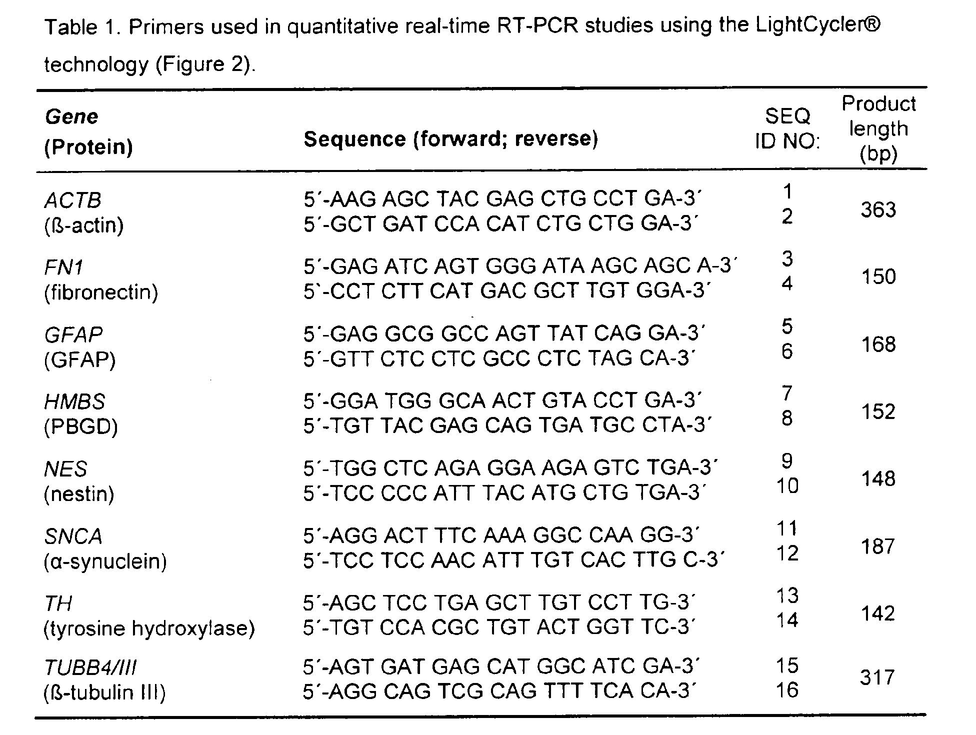

**Electrophysiology.** Cells were investigated for membrane currents between days 10 and 20 after differentiation using the standard whole cell patch clamp technique with an EPC-7 amplifier (List electronics, Heidelberg, Germany) and pClamp data acquisition (Axon Instruments). For better sealing we added 10% FCS 3-5 days prior to patch-clamp experiments. Bathing solution (in mM): 100 NaCl, 54 KCI, 2 CaCl₂, 2 MgCl₂, 10 Hepes, 10 D-Glucose. Pipette solution: 130 KCI, 0.5 CaCl₂, 2 MgCl₂, 5 EGTA, 10 Hepes, 3 Na-ATP. Borosilicate pipettes had resistances of 3-4 MΩ. Seal resistances in the whole cell mode were between 0.1 and 1 GΩ. Data were analysed using pClamp 8.0, Microsoft Excel 97 and Origin 5.0 software.

**Reverse-phase HPLC with electrochemical detection.** For determination of dopamine production and release, media were supplemented with 100 µmol I⁻¹ tetrahydrobiopterin and 200 µmol I⁻¹ ascorbate 3 days prior to medium harvest. Dopamine levels were determined in medium and extracellular buffer stabilized with EGTA/glutathione solution as reported previously [Storch et al., 2001], and stored at-80°C until analysis. Aluminum absorption and HPLC analysis of dopamine have been described [Storch et al., 2001].

**Cell counting and statistics.** For quantification of the percentage of cells producing a given marker, in any given experiments the number of positive cells of the whole well surface was determined relative to the total number of DAPI-labeled nuclei. In a typical experiment, a total of 500 to 1,000 cells were counted per marker. Statistical comparisons were made by Dunnett's *t*-test. If data were not normally distributed, a non-parametric test (Mann-Whitney U-test) was used for comparisons of results. All data are expressed as mean ± s.e.m.

### Supplementary Methods

**Cell culture.** In order to differentiate MSCs and hMNSCs into osteoblasts, we used standard protocols described earlier (Fiedler et al., 2002; Reyes et al., 2001). Briefly, differentiation was induced by plating the cells at 2×10⁴ cells cm⁻² in DMEM medium containing 10% FCS, 1% glutamine and 1% penicillin/streptomycine (all from Biochrom Seromed, Berlin, Germany). We used both cell types from passage 6 to 10. For osteogenic differentiation the medium was supplemented with 0.1 µmol I⁻¹ dexamethasone, 50 mg ml⁻¹ ascorbic acid, and 2.16 mg ml⁻¹ β-glycerophosphate (all from Sigma, St. Louis, MO). Cells were cultured for 10 days and medium was changed twice a week.

**lmmunocytochemistry.** For visualizing osteoblasts, the cultures were analyzed by enzymatic testing for alkaline phosphatase (AP) activity according to standard protocols using an AP staining kit (Sigma) (Fiedler et al., 2002). For quantification of the percentage of cells producing AP, the number of positive cells of the whole well surface was determined relative to the total number of cells under a phase-contrast microscope. In a typical experiment, a total of 500 to 1,000 cells were counted per marker. Statistical comparisons were made by Dunnett's *t*-test. Data were expressed as mean ± s.e.m.

**RNA extraction and RT-PCR analysis.** The differentiation status of hMSCs and hMNSCs was investigated by RT-PCR for the expression of the following osteogenic markers: alkaline phosphatase (AP), runt-related transcription factor 2 (RUNX2), and the transcription factors SOX9 and c-Fos. Glyceraldehyde-3-phosphate dehydrogenase (GAPDH) were used to verify the PCR results as internal standards. Total cellular RNA was prepared using RNAeasy total RNA purification kit and reverse transcription was done with Omniscript RT Kit (all from QIAGEN, Hilden, Germany). PCR reactions were performed essentially as described previously (Fiedler et al., 2002).

Primer sequences (forward, reverse) and lengths of the amplified products were as follows:

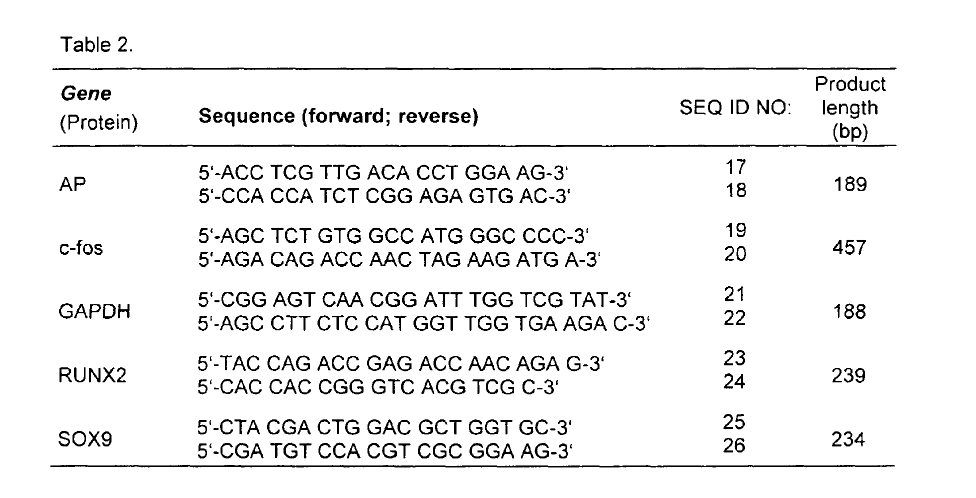

### Results and discussion

To convert adult hMSCs into cells with characteristics of NSCs, we detached hMSCs after 2 to 10 passages (≈10-50 population doublings) and cultured them in uncoated flasks in serum-free P4-8F medium supplemented with epidermal growth factor (EGF) and fibroblast growth factor 2 (FGF-2). The cells did not adhere to the surface of tissue culture flask. One third (37±17%, n=5) of hMSCs died after 3 days, but after 10 to 14 days, the remaining cells formed small spheres of floating cells (Figure 1a,b). The phenotype of these hMNSCs was CD15, CD34, CD45, CD133, and CD166 negative; hMNSCs expressed low levels of CD9 and fibronectin, and higher levels of CD90 and nestin, an intermediate filament protein present in CNS stem cells (Figure 1a,c) [Cattaneo & McKay, 1990; Reynolds & Weiss, 1992; Vogel et al., 2003]. hMNSCs proliferated in vitro for 2 to 10 weeks (≈5-30 population doublings; Figure 1b) without changing morphology and phenotype. The hMNSC phenotype was similar to that of human neural progenitor cells derived from fetal forebrain [Vogel et al., 2003; Uchida et al., 2000], but different from that of hMSC (CD9⁺, CD15⁻, CD34⁻, CD45⁻, CD90^{low/+}, CD166⁺, CD133⁻, fibronectin⁺and nestin^{-/low} (Figure 1a,c) [Fiedler et al., 2002; Vogel et al., 2003]. Notably, CD133 expression was only detected on a small hMNSCs subset (< 1%), consistent with previous reports on neural progenitors [Vogel et al., 2003; Uchida et al., 2000]. Furthermore, we used quantitative real-time RT-PCR to investigate the expression pattern of several mesenchymal and neural genes in hMSCs, hMNSCs and neuronally differentiated hMNSCs (Figure 2, see also Figure 4). Thus, we demonstrated that the high level expression of the FN1 gene, encoding for fibronectin, in hMSCs is virtually lost in hMNSCs following their in vitro differentiation into glia and neurons (Figure 2). Moreover, quantitative analysis of NES (encoding for nestin) showed that hMSCs almost lack while hMNSCs displayed dramatic increase of NES mRNA (Figure 2).

We differentiated hMNSCs in vitro using a glial and a neuronal induction protocol, respectively, by plating the cells onto poly-L-Lysine and adding cytokines and growth factors known to induce differentiation of NSCs into mature glial or neuronal cells (Figure 3a,b). Differentiation into astroglial and oligodendroglial cells required plating of hMNSCs in medium without EGF and FGF-2, but with platelet-derived growth factor (PDGF-BB) and retinoic acid (glial induction medium). After 10 to 14 days, 45±4% (n=3) of hMNSCs acquired morphological and phenotypic characteristics of astrocytes (glial fibrillary acidic protein (GFAP)⁺), 27±5% of oligodendrocytes (galactocerebrosidase (GaIC)⁺), while only a few cells exhibited the early neuronal marker class III β-tubulin (Figure 3b). No mature MAP2ab⁺ neurons could be detected. Electrophysiological analysis of hMNSCs, that were differentiated using the glial induction protocol, revealed that 40% of cells (19 out of 47 recorded cells) showed a sustained outward current of a few hundred pA up to 6 nA. These currents had a voltage-dependent activation and kinetics characteristic for delayed rectifier K⁺ channels (Figure 3d). In a few cells, we could identify small inward currents with voltage dependence and kinetics typical for voltage-activated Na⁺ channels (Figure 3e). Such currents have been described in developing and adult glial cells [Kressin et al., 1995].

Using the neuronal induction medium containing brain-derived neurotrophic factor (BDNF) and retinoic acid, we obtained 42±6% of hMNSCs with early neuronal characteristics (class III β-tubulin expression) and 6±2% expressing the marker molecule for mature neurons, MAP2ab, but only 13±4% of cells with GFAP expression and astroglial morphology (Figure 3a,b). In line with these immunocytochemical results, the expression of the glial gene GFAP as well as the neuronal genes TUBB4/III (encoding for β-tubulin III) and SNCA (α-synuclein) was significantly up-regulated during the neuronal differentiation of hMNSCs (Figure 2). MAP2ab and GFAP were never found in the same cell. Interestingly, in these cultures 11±7% of cells expressed the catecholaminergic marker tyrosine hydroxylase (TH; n=3), which is the rate-limiting enzyme for dopamine synthesis. Consistently, we were able to show up-regulation of TH gene expression (Figure 2) as well as dopamine production and potassium-dependent release in cultures of differentiated hMNSCs (Figure 3f). Unfortunately, patch-clamp experiments to demonstrate neuronal characteristics in these cells were not successful, since addition of 10% FCS was necessary to obtain sufficiently high seal resistances, but this led to an extensive overgrowth of glial cells. However, the differentiation ability of hMNSCs was similar to that of NSCs derived from fetal and adult brain of various species [Cattaneo & McKay, 1990; Song et al., 2002; Carvey et al., 2001; Kilpatrick & Bartlett, 1993; Uchida et al., 2000; Reynold & Weiss, 1992]. In contrast, differentiation of hMSCs using the neuronal induction protocol did not produce mature MAP2ab⁺ neurons or TH⁺/dopamine producing cells (n=5), and did not lead to up-regulation of TUBB4/III or GFAP gene expression (data not shown).

To determine whether individual hMNSCs could generate both neurons and glia, juvenile spheres or MSCs were dissociated and individual cells were isolated by limiting dilution into ultra low attachment multiwell plates; single cells were cultured for 3 to 6 weeks. 11% of the single cells proliferated to generate neurospheres (Figure 3c). Clonal cells were then differentiated for 10 days. Double immunostaining revealed that individual cells in these clones acquired morphologic and phenotypic characteristics of astrocytes (GFAP⁺) and neurons (β-tubulin III⁺) (Figure 3c).

We next tested whether hMNSCs were still able to differentiate into mesenchymal cell types in vitro. We therefore compared the ability of hMSCs and hMNSCs to differentiate into osteoblasts using standard protocols and high density cultures (for Methods, see Supplementary Methods) [Fiedler et al., 2002; Reyes et al., 2001]. After 14 days, 60±3% of hMSCs, but only 17±5% of hMNSCs acquired morphological and phenotypic characteristics of osteoblasts (alkaline phosphatase (AP)⁺; n=3; P<0.01, t-test). Consistently, expression levels of osteogenic marker genes were very low or absent in differentiated hMNSCs (for details, see Figure 5).

The central finding of this invention is that cells with major characteristics of NSCs can be generated with high efficiency from hMSCs, e.g., adult hMSCs, similar to the production of NSCs from embryonic stem cells [Bain et al., 1995; Lee et al., 2000]. The presented human mesodermal-derived NSCs are clonogenic, self-renewing cells that maintain the capacity to differentiate into mature functional brain-specific cell types while dramatically diminishing their mesenchymal differentiation potential, and therefore fulfill all major criteria of NSCs. Thus, we provide proof of conversion of human MSCs into NSCs. However, future experiments are warranted to determine whether reprogramming of a committed mesenchymal stem cell (transdifferentiation) or proliferation and differentiation of a more pluripotent stem cell already harbored in the hMSC suspension is responsible for the cell type conversion in our system [Sanchez-Ramos, 2002; Jiang et al., 2002]. In a typical experiment, for every 1×10⁶ of hMSCs, we routinely obtained 1.6×10⁸ mesodermal-derived NSCs, 0.1×10⁸ neurons, 0.7×10⁸ astroglial, and 0.4×10⁸ oligodendroglial cells. Numerically, at least 7×10⁷ hMNSCs, or 0.5×10⁷ neurons, 3×10⁷ astroglial and 2×10⁷ oligodendroglial cells, respectively, could be produced from one hMSC harvested from the bone marrow sample. This represents the total sum of all parameters of proliferation, cell death, and selective differentiation throughout the multiple conversion and differentiation steps. These cell quantities are sufficient for most transplantation protocols as well as for extensive characterizations of adult NSCs by molecular biology or protein biochemistry methods, such as gene array analysis or proteomics. Thus, hMNSCs could facilitate understanding the molecular mechanisms of neural differentiation in adult human NSCs. Another advantage compared to previous protocols for the conversion of MSCs into neural cells [Sanchez-Ramos et al., 2000; Woodbury et al., 2000] is the possibility to generate undifferentiated NSCs or premature neural cells. These cell types are commonly used and more suitable for neurotransplantation strategies compared to fully differentiated neural cells [Kim et al., 2002; Pluchino et al., 2003; Carvey et al., 2001; Björklund & Lindvall, 2000], since differentiated neuronal cells are well known to poorly survive detachment and subsequent transplantation procedures. The data presented herein demonstrate that hMNSC differentiate into mature neural cells showing functional properties, such as expression of outward-rectifying potassium channels and sodium channels in glial cells as well as dopamine production and potassium-dependent release. Indeed, the method of the invention provides for the first time the possibility to extensively study autologous approaches in neurotransplantation using adult human NSCs.

### References

1. Bain, G.. *et al.* Embryonic stem cells express neuronal properties *in vitro. Dev. Biol.* **168,** 342-357 (1995).
2. Björklund, A., Lindvall, O. Cell replacement therapies for central nervous system disorders. *Nat. Neurosci.* **3,** 537-544 (2000)
3. Carvey, P.M. *et al.* A clonal line of mesencephalic progenitor cells converted to dopamine neurons by hematopoietic cytokines: A source of cells for transplantation in Parkinson's disease, *Exp. Neurol.* **171,** 98-108 (2001).
4. Cattaneo, E., McKay, R. Proliferation and differentiation of neuronal stem cells regulated by nerve growth factor. *Nature* **347,** 762-765 (1990).
5. Fiedler, J. *et al.* BMP-2, BMP-4, and PDGFbb stimulate chemotactic migration of primary human mesenchymal progenitor cells. *J. Cell. Biochem.* **87,** 305-312 (2002).
6. Fricker, R.A. *et al.* Site-specific migration and neuronal differentiation of human neural progenitor cells after transplantation in the adult rat brain. *J. Neurosci.* **19,** 5990-6005 (1999).
7. Friedenstein, A.J., Gorskala U.F., Kulagina, N.N. Fibroblast precursors in normal and irradiated mouse hematopoietic organs. *Exp. Hematol.* **4,** 267-274 (1976).
8. Jiang, Y. *et al.* Pluripotency of mesenchymal stem cells derived from adult marrow. *Nature* **418,** 41-49 (2002).
9. Kilpatrick, T.J., Bartlett, P.F. Cloning and growth of multipotential neural precursors: requirements for proliferation and differentiation. *Neuron* **10,** 255-265 (1993).
10. Kim, J.-H. *et al.* Dopamine neurons derived from embryonic stem cells function in an animal model of Parkinson's disease. *Nature* **418,** 50-56 (2002).
11. Kressin K, Kuprijanova E, Jabs R, Seifert G, Steinhäuser C. Developmental regulation of Na⁺ and K⁺ conductances in glial cells of mouse hippocampal brain slices. *Glia* **15,** 173-187 (1995).
12. Lee, S.-H. *et al.* Efficient generation of midbrain and hindbrain neurons from mouse embryonic stem cells. *Nat. Biotech.* **18,** 675-679 (2000).
13. Mezey, E. *et al.* Turning blood into brain: cells bearing neuronal antigens generated *in vivo* from bone marrow. *Science* **290,** 1779-1782 (2000).
14. Pluchino, S. *et al.* Injection of adult neurospheres induces recovery in a chronic model of multiple sclerosis. *Nature* **422,** 688-694 (2003).
15. Prockop, D.J. Marrow stromal cells as stem cells for non-hematopoietic tissues *Science* **276,** 71-74 (1997).
16. Reyes, M. *et al.* Purification and ex vivo expansion of postnatal human marrow mesodermal progenitor cells. *Blood* **98,** 2615-2525 (2001).
17. Reynolds, B. A., Weiss, S. A. Generation of neurons and astrocytes from isolated cells of the adult mammalian central nervous system. *Science* **255,** 1707-1710 (1992).
18. Sanchez-Ramos, J.R. Neural cells derived from adult bone marrow and umbilical cord blood. *J. Neurosci. Res.* **69,** 880-893 (2002).
19. Sanchez-Ramos, J. *et al.* Adult bone marrow stromal cells differentiate into neural cells in vitro. *Exp. Neurol.* **164**, 247-256 (2000).
20. Song, H., Stevens, C.F., Gage, F.H. Astroglia induce neurogenesis from adult neural stem cells. *Nature* **417,** 39-44 (2002).
21. Sekiya, I. *et al.* Expansion of human adult stem cells from bone marrow stroma: Conditions that maximize the yields of early progenitors and evaluate their quality. *Stem Cells* **20,** 530-541 (2002).
22. Storch, A. *et al.* Long-term proliferation and dopaminergic differentiation of human mesencephalic neural precursor cells. *Exp. Neurol.* **170,** 317-325 (2001).
23. Vogel, W. *et al.* Heterogeneity among human bone marrow-derived mesenchymal stem cells and neural progenitor cells. *J. Haematol.* **88,** 126-133 (2003).
24. Woodbury, D. *et al.* Adult rat and human bone marrow stromal cells differentiate into neurons. *J. Neurosci. Res.* **61,** 364-370 (2000).
25. Uchida, N. *et al.* Direct isolation of human central nervous system stem cells. *Proc. Natl. Acad. Sci. USA* **97,** 14720-14725 (2000).
26. Zhao, L.-R. *et al*. Human bone marrow stem cells exhibit neural phenotypes and ameliorate neurological deficits after grafting into ischemic brain of rats. *Exp. Neurol.* **174,** 11-20 (2002).
27. Bruder, S. P., Jaiswal, N., and Haynesworth, S. E. (1997). Growth kinetics, self-renewal, and the osteogenic potential of purified human mesenchymal stem cells during extensive subcultivation and following cryopreservation. J Cell Biochem *64*, 278-294.
28. Ashjian, P. H., Elbarbary, A. S., Edmonds, B., DeUgarte, D., Zhu, M., Zuk, P. A., Lorenz, H. P., Benhaim, P., and Hedrick, M. H. (2003). In vitro differentiation of human processed lipoaspirate cells into early neural progenitors. Plast Reconstr Surg *111,* 1922-1931.
29. De Bari, C., Dell'Accio, F., Tylzanowski, P., and Luyten, F. P. (2001). Multipotent mesenchymal stem cells from adult human synovial membrane. Arthritis Rheum *44*, 1928-1942.
30. Erices, A., Conget, P., and Minguell, J. J. (2000). Mesenchymal progenitor cells in human umbilical cord blood. Br J Haematol *109*, 235-242.
31. Romanov, Y. A., Svintsitskaya, V. A., and Smirnov, V. N. (2003). Searching for Alternative Sources of Postnatal Human Mesenchymal Stem Cells: Candidate MSC-Like Cells from Umbilical Cord. Stem Cells *21*, 105-110.
32. Tholpady, S. S., Katz, A. J., and Ogle, R. C. (2003). Mesenchymal stem cells from rat visceral fat exhibit multipotential differentiation in vitro. Anat Rec *272A*, 398-402.
33. Wickham, M. Q., Erickson, G. R., Gimble, J. M., Vail, T. P., and Guilak, F. (2003). Multipotent stromal cells derived from the infrapatellar fat pad of the knee. Clin Orthop, 196-212.

## Claims

1. A method of generating neural stem cells, comprising culturing mesenchymal stem cells in a medium containing EGF and FGF-2 wherein hepatocyte growth factor is not present in said medium, and under an atmosphere having an oxygen concentration of less than 20% (v/v).

2. A method according to claim 1 wherein the mesenchymal stem cells are derived from a tissue selected from the group consisting of adult bone marrow, umbilical cord blood and adult fat tissue.

3. A method according to claim 1 or 2 wherein the mesenchymal stem cells are selected from the group consisting of adult bone marrow cells and umbilical cord blood cells.

4. A method according to any one of claims 1 to 3 wherein the mesenchymal stem cells are cultured in the presence of EGF and FGF-2 for at least 10 days.

5. A method according to any one of claims 1 to 4 wherein the mesenchymal stem cells are cultured under an atmosphere having an oxygen concentration of less than 15% (v/v).

6. A method according to any one of claims 1 to 5 wherein the mesenchymal stem cells are cultured under conditions such that no substantial differentiation into neural cells occurs.

7. A method according to any one of claims 1 to 6 wherein the mesenchymal stem cells are cultured under conditions such that a population of cells expressing NES is obtained.

8. A method according to any one of claims 1 to 7 wherein the mesenchymal stem cells are cultured under conditions such that a population of cells expressing *FN1* is obtained.

9. A method for making a medicament comprising the steps of
(a) generating neural stem cells using the method according to any one of claims 1 to 8; and
(b) adding to the cells obtained in step (a) a pharmaceutically acceptable carrier or diluent.

10. A population of mesodermal-derived neural stem cells obtainable by a method according to any one of claims 1 to 8.

11. A population of mesodermal-derived neural stem cells **characterized in that** the neural stem cells express *NES* and *FN1.*

12. A population of mesodermal-derived neural stem cells according to claim 10 or 11 wherein the neural stem cells substantially lack expression of the marker molecules CD15, CD34, CD45, CD133, and CD166.

13. A population of mesodermal-derived neural stem cells according to any one of claims 10 to 12 wherein
(i) the amount of *NES* expression in the neural stem cells is increased by at least 200% compared to the amount of *NES* expression in bone marrow stromal cells, and
(ii) the amount of *FN1* expression in the neural stem cells is 50% to 150 % of the amount of *FN1* expression in bone marrow stromal cells.

14. The use of a population of mesodermal-derived neural stem cells according to any one of claims 10 to 13 for the manufacture of a medicament for the treatment of a neurological disorder.

15. The use according to claim 14 wherein the mesodermal-derived neural stem cells are transplanted to the brain of a patient.

16. The use according to claim 14 or 15 wherein the mesodermal-derived neural stem cells are autologous cells.
